# EUROPEAN PATENT SPECIFICATION

(11) **EP 2 362 916 B1**
(45) Date of publication and mention of the grant of the patent: **23.04.2014**
(21) Application number: 09784057.3
(22) Date of filing: 12.11.2009
(51) Int. Cl.: C12Q 1/70

(54) **HPV TYPES AND VARIANTS ASSOCIATED WITH CERVICAL CANCER AND THE USES THEREOF**
MIT ZERVIXKARZINOM ASSOZIIERTE HPV-TYPEN UND VARIANTEN UND VERWENDUNGEN DAFÜR
TYPES ET VARIANTS DU HPV ASSOCIÉS AU CANCER CERVICAL ET LEURS UTILISATIONS

(30) Priority: 13.11.2008 GB 0820822
(43) Date of publication of application: 07.09.2011
(73) Proprietor: DDL Diagnostic Laboratory B.V., 2288 ER Rijswijk (NL); Institut Catala D'Oncologia, 08907 L'Hospitalet de Llobregat, Barcelona (ES)
(72) Inventor: QUINT, Wilhelmus Gregorius Vincentius, NL-2275 CX Voorburg (NL); DE KONING, Maurits Nicolaas Cornelis, NL-2275 CX Voorburg (NL); GERAETS, Daan, NL-2275 CX Voorburg (NL); VAN DOORN, Leendert Jan, NL-2275 CX Voorburg (NL); DE SANJOSE LLONGUERAS, Silvia, Barcelona (ES); BOSCH, Francisco Javier, Barcelona (ES)
(74) Representative: Hollywood, Jane Constance
(86) International application number: PCT/EP2009/065078
(87) International publication number: WO 2010/055109

(56) References cited:
- WO-A1-2008/017162
- WO-A1-2008/112125
- WO-A2-2005/001137
- KR-A- 20050 017 703
- US-A- 5 656 423
- US-A- 5 712 092
- US-A1- 2007 031 827
- BERGERON C ET AL: "Human papillomaviruses associated with cervical intraepithelial neoplasia. Great diversity and distinct distribution in low- and high-grade lesions." THE AMERICAN JOURNAL OF SURGICAL PATHOLOGY JUL 1992 LNKD- PUBMED:1326896, vol. 16, no. 7, July 1992 (1992-07), pages 641-649, XP009020964 ISSN: 0147-5185
- CHOI YOO-DUK ET AL: "Detection of HPV genotypes in cervical lesions by the HPV DNA Chip and sequencing." GYNECOLOGIC ONCOLOGY SEP 2005 LNKD- PUBMED:16024065, vol. 98, no. 3, September 2005 (2005-09), pages 369-375, XP002586085 ISSN: 0090-8258
- CHAN S Y ET AL: "Analysis of genomic sequences of 95 papillomavirus types: uniting typing, phylogeny, and taxonomy." JOURNAL OF VIROLOGY MAY 1995 LNKD- PUBMED:7707535, vol. 69, no. 5, May 1995 (1995-05), pages 3074-3083, XP002586086 ISSN: 0022-538X
- MALIK ZAINAB A ET AL: "Predictors of seropositivity to human papillomavirus type 53: one of the most prevalent high risk-related cervical human papillomaviruses." VIRAL IMMUNOLOGY SEP 2008 LNKD- PUBMED:18681800, vol. 21, no. 3, September 2008 (2008-09), pages 371-377, XP002598898 ISSN: 1557-8976
- TORNESELLO MARIA LINA ET AL: "Prevalence of alpha-papillomavirus genotypes in cervical squamous intraepithelial lesions and invasive cervical carcinoma in the Italian population." JOURNAL OF MEDICAL VIROLOGY DEC 2006 LNKD- PUBMED:17063505, vol. 78, no. 12, December 2006 (2006-12), pages 1663-1672, XP002598899 ISSN: 0146-6615
- SCHMITT MARKUS ET AL: "Homogeneous amplification of genital human alpha papillomaviruses by PCR using novel broad-spectrum GP5+ and GP6+ primers." JOURNAL OF CLINICAL MICROBIOLOGY MAR 2008 LNKD- PUBMED:18199790, vol. 46, no. 3, March 2008 (2008-03), pages 1050-1059, XP002598900 ISSN: 1098-660X
- VÁRNAI ALINDA D ET AL: "The spectrum of cervical diseases induced by low-risk and undefined-risk HPVS: implications for patient management." ANTICANCER RESEARCH 2007 JAN-FEB LNKD- PUBMED:17348442, vol. 27, no. 1B, January 2007 (2007-01), pages 563-570, XP009138103 ISSN: 0250-7005
- HUANG H J ET AL: "Human papillomavirus genotyping by a polymerase chain reaction-based genechip method in cervical carcinoma treated with neoadjuvant chemotherapy plus radical surgery." INTERNATIONAL JOURNAL OF GYNECOLOGICAL CANCER : OFFICIAL JOURNAL OF THE INTERNATIONAL GYNECOLOGICAL CANCER SOCIETY 2004 JUL-AUG LNKD- PUBMED:15304160, vol. 14, no. 4, July 2004 (2004-07), pages 639-649, XP002598902 ISSN: 1048-891X

## Description

The present invention relates to the field of detection and identification of Human Papillomavirus (HPV) infections, particularly the identification of HPV type 69 associated with cervical cancer.

### BACKGROUND OF THE INVENTION

Cervical cancer is the second most common malignancy in women; following breast cancer. Carcinoma of the cervix is unique in that it is the first major solid tumor in which HPV DNA is found in virtually all cases and in precursor lesions worldwide.

Over 100 HPV types have been characterized and are numbered in chronological order of isolation. HPV is epitheliotropic and infects only the skin (cutaneous types) or the mucosa of the respiratory and anogenital tract (mucosal types). More than 40 HPV types are known to infect the uterine cervix. Based on the induced benign, premalignant or malignant lesions, HPV is divided into low-risk (e.g., HPV types 6, 11, 42, 43 and 44) and high-risk types (e.g., types 16, 18, 31, 33 and 45), respectively. The high-risk types account for more than 99% of all invasive cervical cancers. Consequently, detection and identification of HPV types is very important. The high-risk types are by definition consistently found in high grade SIL (Squamous Intraepithelial Lesion) and carcinoma in-situ whereas low risk types are mainly found in low grade SIL. This epidemiological observation is supported by molecular findings. For instance, the E6 and E7 proteins from low-risk types 6 and 11 bind p53 and pRB too weakly to immortalize keratinocytes *in vitro* or to induce malignant transformation in vivo. The circular ds-DNA genome of low-risk HPV types remains episomal whereas the genome of high-risk HPV types is able to integrate into the human genome.

Screening for malignant and premalignant disorders of the cervix is usually performed according to the Papanicoloau (PAP) system. The cervical smears are examined by light microscopy and the specimens containing morphologically abnormal cells are classified into PAP I to V, at a scale of increasing severity of the lesion. This cytomorphological method is an indirect method and measures the possible outcome of an HPV infection. Therefore, HPV DNA detection and typing is of importance in secondary screening in order to select patients for monitoring (follow-up) and treatment. This means that cervical smears classified as PAP II (atypical squamous metaplasia) or higher classes should be analyzed for low-risk and high risk HPV types. Follow-up studies have shown that only high-risk HPV types are involved in the progression from cytologically normal cervix cells to high grade SIL. These results indicate that the presence of high-risk HPV types is a prognostic marker for development and detection of cervical cancer.

Diagnosis of HPV by culture is not possible. Also diagnosis by detection of HPV antibodies appears to be hampered by insufficient sensitivity and specificity. Direct methods to diagnose an HPV infection are mainly based on detection of the viral DNA genome by different formats of DNA/DNA or RNA/DNA hybridization with or without prior amplification of HPV DNA. The polymerase chain reaction (PCR) is a method that is highly efficient for amplification of minute amounts of target DNA. Nowadays, mainly three different primer pairs are used for universal amplification of HPV DNA ("broad spectrum primers"). Three of these primer pairs, MY11/MY09, GP5/GP6 and the SPF10 system, are directed to conserved regions among different HPV types in the L1 region (Manos et al., ,Cancer Cells 1989, 7:209-214; Van den Brule et al., J Clin Microbiol. 1990 28(12):2739-43, WO9914377). The PGMY system, a modification of the MY09/11 is also used (see Gravitt, P., 2000. J. Clin. Microbiol. 38:357-361). Another primer pair, CPI/CPII, is directed to conserved regions in the E1 region but is not often used. WO2006077102 discloses a further set of suitable primers.

There are several methods to identify the various HPV types.

HPV DNA can be typed by PCR primers that recognize only one specific type. This method is known as type-specific PCR. Such methods have been described for HPV types 6, 11, 16, 18, 31 and 33 The primers are aimed at the E5, L1, E6, L1, E2 and E1 regions of the HPV genome for types 6, 11, 16, 18, 31 and 33, respectively.

Another method is general amplification of a genomic part from all HPV types followed by hybridization with two cocktails of type-specific probes differentiating between the oncogenic and non-oncogenic groups, respectively. A similar typing method has been described without prior amplification of HPV DNA. In the hybrid capture assay (*digene* HPV hc2 Test; Qiagen), each sample is tested for a group of "high-risk" HPV types (i.e., 16, 18, 31, 33, 35, 39, 45, 51, 52, 56, 58, 59 and 68) and for another group of "low-risk" HPV types (i.e., 6, 11, 42, 43 and 44) (Cox et al., Am J Obstet Gynecol. 1995 Mar;172(3):946-54.).

A detection and typing system disclosed in WO9914377 utilises a PCR amplification step and a reverse line blot hybridization with type specific probes.

At present, formal classification of human papillomaviruses is based on sequence analysis of a 291 bp fragment from the L1 region (Chan et al. J Virol. 1995 May:69(5):3074-83. De Villiers et al., Virology. 2004 Jun 20;324(I): 17-27) Phylogenetic analysis of these sequences allows classification of the different HPV types. By definition, if the sequence difference across this region between two HPV isolates is higher than 10% they are classified as different types. Consequently, if the sequence differs more than 10% from any known HPV type it is classified as a novel HPV type. HPV isolates that differ between 2-10% are classified as different subtypes. Finally, if the sequence variation is below 2%, the 2 isolates are classified within the same subtype as different variants.

HPV sequences of HPV types (16, 18, 31, 33, 35, 39, 45, 51, 52, 56, 58, 59, 68, 73 and 82 [Bosch et al., Dis Markers. 2007:23(4):213-27] have already been reported to be present as a single infection in cervical carcinomas and are therefore classified as oncogenic types. However the carcinogenic potential of certain HPV types is still not confirmed.

### Statement of the invention

The invention is as set out in the claims.

Described herein is a probe capable of specific hybridization with an HPV viral polynucleotide target possibly present in a biological sample,
the HPV viral polynucleotide target comprising a polynucleotide sequence of any of SEQ ID NO 1 - SEQ ID NO 30, or fragment thereof, that comprises a discriminatory nucleotide, as indicated in Table 1,
the probe being capable of specific hybridisation to the target over at least the discriminatory nucleotide position, to allow the target sequence to be distinguished from other HPV viruses types.

Further described is a method of typing any HPV nucleic acid possibly present in a biological sample comprising the steps of contacting any such nucleic acid with at least one probe of the invention under stringent conditions, and analysing HPV type based upon the hybridisation result so obtained.

Further described is a kit comprising a probe of the present invention in combination with one or more of the following:
1) a second probe of the present invention;
2) a probe capable of specific hybridization with an HPV viral polynucleotide target, such as a probe for a known high risk HPV type, the types including for example HPV 16, 18, 31, 33, 35, 39, 45, 51 , 52, 56, 58, 59, 68, 73 and 82;
3) a PCR primer suitable for amplification of HPV DNA;
4) an enzyme for amplification of an HPV polynucleotide; or
5) a solid support

Described herein is an isolated HPV polynucleotide sequence distinguishable from other HPV types, or subtypes or variants by the presence of one or more discriminatory nucleotides listed in table 1, or a complementary polynucleotide sequence thereto.

Described herein is an isolated HPV polynucleotide sequence comprising a discriminatory nucleotide as indicated in table 1, wherein the polynucleotide encodes all or a fragment of the HPV L1 protein, or a fragment thereof.

Described herein is an isolated HPV polynucleotide sequence of the invention which comprises the complete genomic DNA of an HPV virus.

Described herein is an isolated polynucleotide sequence comprising the complete sequence of any of the sequences of SEQ ID NO 1 - SEQ ID NO 30, or a complementary polynucleotide sequence thereto.

Described herein is an HPV virus comprising a polynucleotide sequence of the sequences of any of SEQ ID NO 1 - SEQ ID NO 30.

Described herein is an HPV Virus-Like-Particle (VLP) comprising the HPV L1 protein or fragment thereof expressed from an L1 sequence comprising any of SEQ ID NO 1 - SEQ ID NO 30.

Described herein is a process for identification of HPV viral types associated with cervical cancer, the process comprising:
1) isolation of any HPV viral DNA possibly present in a cervical cancer tissue sample;
2) confirming the presence of HPV DNA in the sample using a detection system which is specific for HPV DNA but not HPV type specific,
3) typing any HPV DNA present using type specific detection with probes against known high risk types, and
4) sequencing the DNA in samples found to be positive for HPV DNA but negative for DNA from known high risk HPV types to identify the sequence of any HPV virus in the sample.

In one aspect the invention relates to an *in vitro* use of a kit comprising a probe to specifically detect HPV type 69 in the screening of tissue samples for the identification of a risk of development of cervical cancer, wherein detection of HPV type 69 is indicative of high risk.

In one aspect the invention relates to an *in vitro* use of a probe specific for HPV type 69 in the screening of tissue samples for the identification of development of cervical cancer, wherein detection of HPV 69 is indicative of high risk.

In one aspect the invention relates to use of HPV type 69 in the preparation of a vaccine for prevention or treatment of cervical cancer.

In one aspect the invention relates to a vaccine for the prevention of cervical cancer comprising an HPV antigen or nucleic acid encoding said antigen, wherein the HPV antigen is HPV type 69.

Also described is a vaccine for the prevention of cervical cancer comprising an HPV Virus-Like-Particle (VLP) comprising the HPV L1 protein, or fragment thereof, expressed from an L1 sequence comprising any of SEQ ID NO 1 - SEQ ID NO 30.

### Figures and Tables

Figure 1 illustrates the algorithm for sample testing.
Figure 2 illustrates a schematic overview of the HPV genome and primer combinations used for sequencing.
Figure 3 illustrates the alignment of novel HPV sequences found in cervical carcinoma.
Table 1 defines SEQ ID 1 - SEQ ID 30.
Table 2 lists the HPV types and variants identified as being associated with cervical cancer.

### Detailed description

Described herein are polynucleotides, such as DNA or RNA, which can serve as probes in the identification of different HPV types, subtypes or variants, or polynucleotide which serve as targets for such probes (for example, as controls in experiments), and to vaccines or viruses comprising said polynucleotide sequences.

Described herein is an isolated HPV polynucleotide sequence distinguishable from other HPV types, or subtypes or variants by the presence of one or more discriminatory nucleotides listed in table 1, or a complementary polynucleotide sequence thereto.

Described herein is an isolated HPV polynucleotide sequence comprising a discriminatory nucleotide as indicated in table 1, wherein the polynucleotide encodes all or a fragment of the HPV L1 protein, or a fragment thereof.

An HPV polynucleotide can be a sequence capable of use in discrimination between different HPV types, subtypes and/or variants.

Described herein is an isolated polynucleotide sequence comprising the complete sequence of any of the sequences of SEQ ID NO 1 - SEQ ID NO 30, or a complementary polynucleotide sequence thereto.

Described herein is:
SEQ ID NO 1 An isolated HPV polynucleotide sequence which comprises a T at position 6621 when compared to HPV 16 reference sequence K02718.
SEQ ID NO 2 An isolated HPV polynucleotide sequence which comprises a C at position 6620 when compared to HPV 16 reference sequence K02718.
SEQ ID NO 3 An isolated HPV polynucleotide sequence which comprises a G at position 6620 when compared to HPV 16 reference sequence K02718.
SEQ ID NO 4 An isolated HPV polynucleotide sequence which comprises a G at position 6620 when compared to HPV 16 reference sequence K02718.
SEQ ID NO 5 An isolated HPV polynucleotide sequence which comprises a T at position 6555 when compared to HPV 18 reference sequence EF202155.
SEQ ID NO 6 An isolated HPV polynucleotide sequence which comprises a G at position 6624 and an A at position 6638 when compared to HPV 39 reference sequence M62849.
SEQ ID NO 7 An isolated HPV polynucleotide sequence which comprises a G at position 6624 when compared to HPV 39 reference sequence M62849.
SEQ ID NO 8 An isolated HPV polynucleotide sequence which comprises a G at position 2598 when compared to HPV 68 reference sequence M73258.
SEQ ID NO 9 An isolated HPV polynucleotide sequence which comprises a G at position 6578 when compared to HPV 56 reference sequence X74483.
SEQ ID NO 10 An isolated HPV polynucleotide sequence which comprises a G at position 6578 when compared to HPV 56 reference sequence X74483.
SEQ ID NO 11 An isolated HPV polynucleotide sequence which comprises a G at position 6578 and a G at position 6595 when compared to HPV 56 reference sequence X74483.
SEQ ID NO 12 An isolated HPV polynucleotide sequence which comprises a G at position 6578 when compared to HPV 56 reference sequence X74483.
SEQ ID NO 13 An isolated HPV polynucleotide sequence which comprises a C at position 6603 when compared to HPV 67 reference sequence D21208.
SEQ ID NO 14 An isolated HPV polynucleotide sequence which comprises a C at position 6603 and a G at position 6697 when compared to HPV 67 reference sequence D21208.
SEQ ID NO 15 An isolated HPV polynucleotide sequence which comprises a G at position 6569 and a C at position 6603 when compared to HPV 67 reference sequence D21208.
SEQ ID NO 16 An isolated HPV polynucleotide sequence which comprises an A at position 6622 when compared to HPV 30 reference sequence X74474.
SEQ ID NO 17 An isolated HPV polynucleotide sequence which comprises a C at position 6559 when compared to HPV 26 reference sequence X74472.
SEQ ID NO 18 An isolated HPV polynucleotide sequence which comprises an A at position 26 and a T at position 40 when compared to HPV 64 reference sequence U 12495.
SEQ ID NO 19 An isolated HPV polynucleotide sequence which comprises an A at position 6539, an A at position 6548, and an A at position 6550 when compared to HPV 54 reference sequence AF436129.
SEQ ID NO 20 An isolated HPV polynucleotide sequence which comprises 33 nucleotides before position 1 when compared to unclassified HPV reference sequence AJ012757.
SEQ ID NO 21 An isolated HPV polynucleotide sequence which comprises a C at position 6689 when compared to HPV 30 reference sequence X74474.
SEQ ID NO 22 An isolated HPV polynucleotide sequence which comprises a G at position 6714 when compared to HPV 30 reference sequence X74474.
SEQ ID NO 23 An isolated HPV polynucleotide sequence which comprises a T at position 6617 when compared to HPV 16 reference sequence K02718.
SEQ ID NO 24 An isolated HPV polynucleotide sequence which comprises an A at position 6469 when compared to HPV 34 reference sequence X74476.
SEQ ID NO 25 An isolated HPV polynucleotide sequence which comprises a T at position 6580 when compared to HPV 54 reference sequence U37488.
SEQ ID NO 26 An isolated HPV polynucleotide sequence which comprises a C at position 21 when compared to HPV 64 reference sequence U 12495.
SEQ ID NO 27 An isolated HPV polynucleotide sequence which comprises a G at position 2598 when compared to HPV 68 reference sequence M73258.
SEQ ID NO 28 An isolated HPV polynucleotide sequence which comprises an A at position 6635 when compared to HPV 30 reference sequence X74474.
SEQ ID NO 29 An isolated HPV polynucleotide sequence which comprises a G at position 6583, a T at position 6590, a T at position 6607 and an A at position 6619 when compared to HPV 82 reference sequence AB027021.
SEQ ID NO 30 An isolated HPV polynucleotide sequence which comprises an A at position 6818 & T at position 6833 when compared to HPV 91 reference sequence AF419318.

Described herein is a probe capable of specific hybridization with an HPV viral polynucleotide target possibly present in a sample, the HPV viral polynucleotide target comprising a polynucleotide sequence of any of SEQ ID NO 1 - SEQ ID NO 30, the probe being capable of specific hybridisation across all or a part of the respective sequence of any of SEQ ID NO 1 - SEQ ID NO 30 to allow the target sequence to be distinguished from other HPV viruses types, subtypes or variants.

The "sample" may be any material which may contain HPV nucleic acid, such as biological material, for example taken either directly from a human being (or animal), or after culturing (enrichment), or may be recombinant HPV nucleic acid expressed in a host cell. Biological material may be e.g. urine, or scrapes/biopsies from the urogenital tract or any part of the human or animal body.

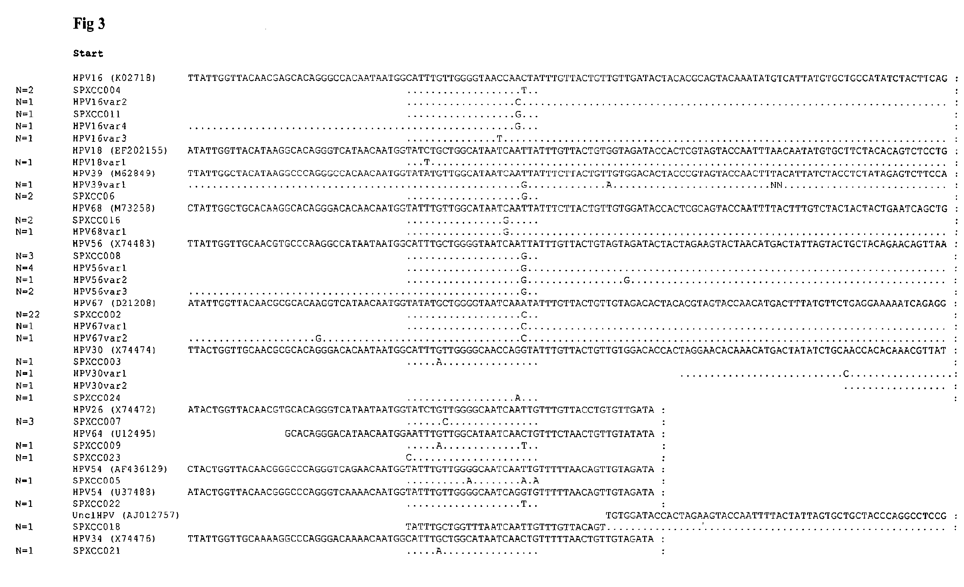

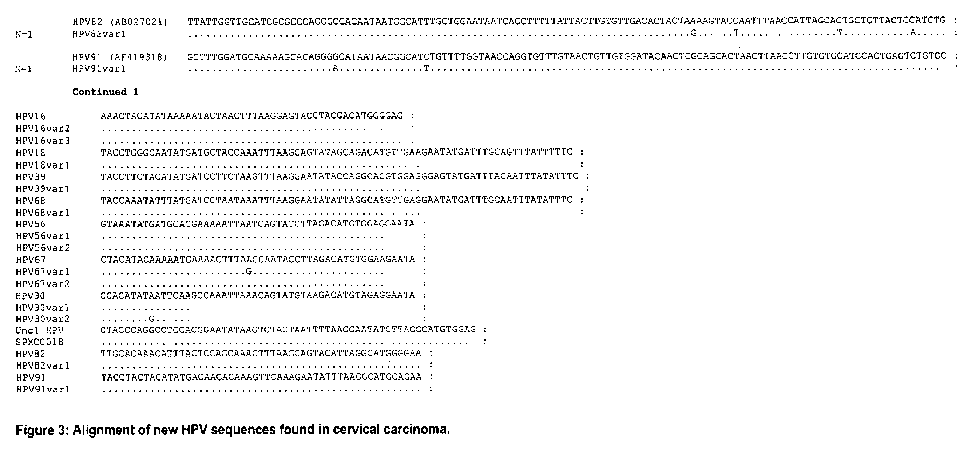

Reference to SEQ ID NO 1 - SEQ ID NO 30 is taken herein to refer to any of the 30 sequences (SEQ ID NO 1, SEQ ID NO 2.... SEQ ID NO 30) disclosed herein in table 1 and Figure 3.

Probes described herein are capable of specific hybridisation across all or a part of the sequence of any of SEQ ID NO 1 - SEQ ID NO 30, to allow a target sequence comprising all or a part of the sequence of SEQ ID NO 1 - SEQ ID NO 30 to be distinguished from other HPV virus types, subtypes or variants. Reference to HPV 'types' hereinafter is taken to include subtypes or variants, unless otherwise apparent from the context, and reference herein to HPV types can be replaced by reference to HPV types, subtypes and variants.

Described herein is a probe capable of distinguishing between HPV viruses at one or more of the specific points of difference between the determined sequences of any of SEQ ID NO 1 - SEQ ID NO 30 and the respective reference sequences noted in the Figures herein.

The term "probe" according to the present invention generally refers to a single-stranded oligonucleotide which is designed to specifically hybridize to HPV polynucleic acids.

The term "target" or "target sequence" of a probe according to the present invention is a sequence within an HPV polynucleic acid to which the probe or the primer is completely complementary or partially complementary (where partially complementary allows for some degree of mismatch). It is to be understood that the complement of said target sequence is also a suitable target sequence in some cases. Probes of the present invention are suitably complementary to at least the central part of their target sequence. In most cases the probes are completely complementary to their target sequence.

"Specific hybridization" of a probe to a region of an HPV polynucleic acid target means that said probe forms a duplex with part of this region or with the entire region under the experimental conditions used, and that under those conditions said probe does not form a duplex with other regions of the polynucleic acids present in the sample to be analysed. It should be understood that probes that are designed for specific hybridisation within a region of HPV polynucleic acid may fall entirely within said region or may to a large extent overlap with said region (i.e. form a duplex with nucleotides outside as well as within said region).

Suitably the specific hybridisation of a probe to a nucleic acid target region occurs under stringent hybridisation conditions, such as 3X SSC, 0.1 % SDS, at 50°C. The skilled person knows how to vary the parameters of temperature, probe length and salt concentration such that specific hybridisation can be achieved. Hybridization and wash conditions are well known and exemplified in Sambrook, et al., Molecular Cloning: A Laboratory Manual, Second Edition, Cold Spring Harbor, N. Y., (1989), particularly Chapter 11 therein. When needed, slight modifications of the probes in length or in sequence can be carried out to maintain the specificity and sensitivity required under the given circumstances.

Preferred stringent conditions are suitably those which allow for a type specific probe binding to only one HPV type. Probes which specifically hybridise to the target suitably are at least 95% complementary to the target sequence over their length, suitably greater than 95% identical such as 96%, 97%, 98%, 99% and most preferably 100% complementary over their length to the target HPV sequence. Suitably each nucleotide of the probe can form a hydrogen bond with its counterpart target nucleotide.

Preferably the complementarity of probe with target is assessed by the degree of A:T and C:G base pairing, such that an adenine nucleotide pairs with a thymine, and such that a guanine nucleotide pairs with a cytosine, or vice versa. In the RNA form, T may be replaced by U (uracil).

Preferably, the probes of the invention are about 5 to 50 nucleotides long, more preferably from about 10 to 25 nucleotides. Particularly preferred lengths of probes include 5, 6, 7, 8, 9, 10, 11, 12, 13, 14, 15, 16, 17, 18, 19, 20, 21, 22, 23, 24 or 25 nucleotides. The nucleotides as used in the present invention may be ribonucleotides, deoxyribonucleotides and modified nucleotides such as inosine or nucleotides containing modified groups which do not essentially alter their hybridization characteristics. Probes may include locked nucleic acid (LNA) nucleotides, which can be mixed with DNA or RNA bases in the oligonucleotide whenever desired (see Kaur, H.; Arora, A.; Wengel, J.; Maiti, S. (2006). "Thermodynamic, Counterion, and Hydration Effects for the Incorporation of Locked Nucleic Acid Nucleotides into DNA Duplexes". Biochemistry 45 (23): 7347-55.

The oligonucleotides used as probes may also comprise nucleotide analogues such as phosphorothiates, alkylphosphorothiates or peptide nucleic acids or may contain intercalating agents. As most other variations or modifications introduced into the original DNA sequences of the invention these variations will necessitate adaptions with respect to the conditions under which the oligonucleotide should be used to obtain the required specificity and sensitivity. However the eventual results of hybridization will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

Probes of the present invention may be labelled in any suitable manner to allow detection of binding to a target. For example the probe may be labelled with a radioactive, luminescent or fluorescent label, or with a label that is capable of reacting with another component to produce a detectable signal which may be, for example, a radioactive, luminescent or fluorescent signal. The nature of the label may be isotopic (³²P,³⁵S, etc.) or non-isotopic (biotin, digoxigenin, etc.) Where nucleic acids are labelled the labelling may be carried out by the use of labelled nucleotides incorporated during any polymerase step of the amplification.

In one aspect the probe of the present invention may be used in combination with other probes to form sets of probes, for example a set for detection of high risk HPV types. The sets of probes of the present invention may include at least 2, 3, 4, 5, 6, 7, 8, 9, 10, 11 , 12, 13, 14, 15, 16, 17, 18, 19, 20, 21 , 22, 23, 24, 25, 26, 27, 28, 29, 30, 31 , 32, 33, 34, 35 , 36 , 37,38, 39, 40, 41 , 42, 43, 44, 45, 46, 47, 48, 49, 50 or more probes.

For the avoidance of doubt the probes of the invention are claimed individually and in groups of (where appropriate) at least 5, 10, 15, 20, 25, 30, 35, 40 probes.

The probe may be immobilised on a solid support. By way of example, a probe may be immobilised on a solid support as described in WO9914377, and optionally used in a line probe assay. The probe may optionally be immobilised in combination with other probes which are specific for HPV types, subtypes or variants.

The term "solid support" can refer to any substrate to which an oligonucleotide probe can be coupled, provided that it retains its hybridization characteristics and provided that the background level of hybridization remains low. Usually the solid substrate will be a microtiter plate (e.g. in the DEIA technique disclosed in WO9914377), a membrane (e.g. nylon or nitrocellulose) or a microsphere (bead) or a chip. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH2 groups, SH groups, carboxylic groups, or coupling with biotin, haptens or proteins.

Where there are multiple probes then said probes may be applied in two or more (possibly as many as there are probes) distinct and known positions on a solid substrate. Often it is preferable to apply two or more probes together in one and the same position of said solid support. Described herein is a solid support attached to 1 or more probes of the present invention.

The probes of the invention can be coupled to the surface of a bead, for example as described in WO2007082881. The use of beads (or microbeads), such as spherical beads also referred to herein as microspheres, in multiplex analysis has been described previously in, for example, Dunbar SA. (Clin Chim Acta. 2006 Jan; 363(I-2):71-82) and through the Luminex product information and website (www.Luminexcorp.com). The Luminex system is a bead-based multiplexing (array) technology which has proven to be very powerful for analyzing multiple parameters or analytes within one sample. It delivers results on many bio-assay formats including nucleic acid assays, receptor-ligand assays, immunoassays and enzymatic assays. The use of liquid bead microarrays for HPV detection is discussed in Wallace J et al, (J Mol Diagn. 2005 Feb; 7(1):72- 80.) Protocols and materials are known and published for Luminex type systems, and are given on the Luminex website in addition to the disclosure on WO2007082881.

Where probes are coupled to beads then hybridisation between a probe and target may take place in a liquid media, and binding of probe to target may be assessed by, for example, flow cytometry.

Detection of binding may also be carried out in the context of a microarray, using for example methods as described in EP373203, EP386229, EP0804731 and EP619321. Such techniques are well known to the person skilled in the art.

Suitable example of probes of the invention include DNA sequences which are complementary to their target sequence at all nucleotide positions within the whole or part sequences of SEQ ID 1 - SEQ ID 30 and which comprise a nucleotide which is indicated in Figure 3 as being the point of difference between the sequence and a known reference sequence. For example, a suitable probe might comprise a sequence complementary to the DNA sequence CATTTGTTGGGGTAACCAATTA for the SPXCC004 sequence, whereas the HPV 16 reference sequence is CATTTGTTGGGGTAACCAACTA.

Probes of the invention are probes against high risk HPV type 69. In one aspect these probes may be used in combination with probes from previously known high risk types.

The probes of the present invention are for use in *in vitro* methods for the identification of HPV types in tissue samples.

Described herein is a method of typing any HPV nucleic acid possibly present in a biological sample comprising the steps of contacting any such nucleic acid with at least one probe of the invention under stringent conditions, and analysing HPV type based upon the hybridisation result so obtained.

Described herein is a method in which an amplification step is carried out to amplify any HPV nucleic acid possibly present in a biological sample prior to the hybridization step. In one aspect the amplification step is non type specific, in that DNA from multiple different HPV types is amplified (also known as broad spectrum PCR). In one aspect any amplified DNA is probed in a non type specific manner to detect the presence of HPV DNA. Such amplification and detection can be carried out using the HPV DEIA technique as described herein and in WO9914377.

Amplification is carried out in one aspect using a primer set which amplifies the 65-base pair fragment from the L1 region of the HPV genome as described in (Kleter et al., J Clin Microbiol. 1999 Aug;37(8):2508-17). In one aspect the SPF₁₀ PCR set is used. Alternatively the primers of the MPF system as disclosed in WO2006077102, GP5+6+ system, or combinations of primers from these different sets may be used to amplify the HPV region of interest.

In one aspect any HPV nucleic acid found in a sample is specifically typed, for example by the use of type specific probes of the invention, optionally in combination with other probes or direct sequencing.

Described herein is a method for detection and/or typing of any HPV nucleic acid possibly present in a biological sample, the method comprising the steps of: (i) amplification of a polynucleic acid fragment comprising a region of any HPV nucleic acid in the sample, and (ii) contacting any amplified fragments from step (i) with at least one probe of the invention capable of specific hybridization within the amplified region of HPV, to determine the HPV type.

Described herein is a process for identification of HPV viral types associated with cervical cancer, the process comprising:
1) isolation of any HPV viral DNA possibly present in a cervical cancer tissue sample;
2) confirming the presence of any HPV DNA in the sample using non-type specific HPV detection,
3) typing any HPV DNA present using type specific detection for known high risk HPV types, and
4) sequencing the DNA in samples found to be positive for HPV DNA but negative for known HPV DNA to identify the sequence of any new HPV virus type in the sample.

The process further optionally comprises designing probes based upon new HPV viral sequences which allow discrimination between these new types and known HPV DNA types.

Sequence comparisons of nucleic acid identity/homology are readily carried out by the skilled person, for example using the BLAST and BLAST 2.0 algorithms, which are described in Altschul et al., Nucl. Acids Res. 25:3389-3402 (1977), and Altschul et al., J. Mol. Biol. 215:403-410 (1990), respectively. BLAST and BLAST 2.0 can be used, for example with the default parameters, to determine percent sequence identity for the polynucleotide of the invention. Software for performing BLAST analyses is publicly available through the National Center for Biotechnology Information.

Described herein is a kit comprising a probe of the present invention in combination with one or more of the following:
1) an additional (different) probe of the present invention;
2) an additional probe capable of specific hybridization with an HPV viral polynucleotide target, such as a probe for a known high risk HPV type selected from, by way of example, HPV 16, 18, 31 , 33, 35, 39, 45, 51, 52, 56, 58, 59, 68, 73 and 82, or a probe for a low risk type;
3) a PCR primer or primer set suitable for amplification of HPV DNA;
4) an enzyme for amplification of an HPV polynucleotide;
5) a solid support

Where there are 2 or more probes these may be immobilised on the same or different supports, and may be labelled or unlabelled.

Primers and probes of components 2) or 3) above may be any suitable primer or probes, such as any of those described in WO9914377, WO03014402, WO2006077102 or WO2007082881.

Other components of a kit may include a hybridization buffer, or components necessary for the production of said buffer, or instructions to prepare said buffer; a wash solution, or components necessary for the production of said solution, or instructions to prepare said solution; a means for detection of the hybrids formed; a means for attaching the probe(s) to a known location on a solid support.

Vaccines against HPV are commercially available and rely upon HPV virus like particles formed from the L1 protein, either full length or truncated. The techniques used to produce HPV L1 in the form of VLPs are well known in the art, for example, as disclosed in WO9302184, WO9400152, WO9420137, WO9913056, WO0142780 and WO961 1272.

HPV vaccines may also comprise an adjuvant, such as adjuvants comprising aluminium, or comprising 3D MPL. Other adjuvants are well known in the art and disclosed in the references above.

HPV viruses comprising the sequence of any of SEQ ID no 1 - SEQ ID no 30 identified herein may be used in the production of an HPV vaccine, the vaccine suitably being in the form of virus like particles that contain all or part of the HPV L1 protein, using known techniques well documented in the art.

Described herein is an HPV Virus-Like-Particle (VLP) comprising an HPV L1 protein, or fragment thereof, expressed from an L1 sequence comprising any of SEQ ID NO 1 - SEQ ID NO 30, or complement thereof.

The present invention has identified HPV type 69 in cervical carcinomas.

HPV type 69 was not previously considered to be high risk. This finding has implications for vaccine design, screening and diagnostics.

Reference to variants of HPV 64, 34 and 54 herein as high risk types is a reference to HPV strains having the same discriminatory mutations as disclosed in table 1.

Reference to these types, as with all HPV types listed herein, includes variants of these types. Variants of a type still fall with the definition of that type, as mentioned above in the background section, but may vary by, for example, 1 , 2, 3, 4, 5, 6, 7, 8, 9, 10, or more, positions from reference HPV sequences, such as the references sequences given herein.

Thus, the invention relates to an *in vitro* use of kits as defined in the claims for identifying the risk of developing cervical cancer, the kits comprising a probe to specifically detect HPV type 69. For the avoidance of doubt probes specific for HPV type 69 may be probes that detect the specific HPV sequences identified in the present invention, or may be probes against other HPV type 69 sequences.

The invention also relates to use of probes as defined in the claims specific for HPV type 69 in the screening of tissue samples for the identification of high risk HPV type 69, and the use of HPV type 69 in the preparation of a vaccine for prevention or treatment of HPV.

In one aspect the invention relates to a vaccine for the prevention of cervical cancer comprising an HPV antigen from HPV type 69, or nucleic acid encoding said antigen. Said HPV antigen may be any suitable antigen, or combination thereof, such as L1, E1, E6 or E7, and in one aspect is the L1 antigen, or immunogenic fragment thereof, or a nucleic acid encoding the same.

For the avoidance of doubt the terms 'comprising', 'comprise' and 'comprises' herein is intended by the inventors to be optionally substitutable with the terms 'consisting of, 'consist of, and 'consists of, respectively, in every instance. Embodiments herein relating to "vaccine compositions" of the invention are also applicable to embodiments relating to "immunogenic compositions" of the invention, and vice versa. The term "about" (or "around") in all numerical values allows for a 5% variation, i.e. a value of about 1.25% would mean from between 1.19%-1.31 %.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the measurement, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, BBC, AAABCCCC, CBBAAA, CABABB, and so forth. The skilled artisan will understand that typically there is no limit on the number of items or terms in any combination, unless otherwise apparent from the context.

All statements made concerning one embodiment of the invention may be combined with other embodiments of the invention, unless apparent from the context or explicitly excluded.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept and scope of the invention. All such similar substitutes and modifications apparent to those skilled in the art are deemed to be within the scope and concept of the invention as defined by the appended claims.

The present invention is now exemplified with reference to the following examples, which are not limiting upon the invention:

### EXAMPLE 1 - detection of novel HPV viral sequences

### Novel HPV sequences in cervical carcinoma with single HPV infections

### Introduction

Identification of the different HPV genotypes in cervical carcinoma specimens is crucial to elucidate the role of the HPV in cervical disease development.

HPV sequences of HPV types (16, 18, 31, 33, 35, 39, 45, 51, 52, 56, 58, 59, 68, 73 and 82 (Bosch et al., Dis Markers. 2007;23(4):213-27)) have already been reported to be present as a single infection in cervical carcinomas and are therefore classified oncogenic types. However the carcinogenic potential of certain HPV types is still under debate.

In order to investigate which HPV genotypes are present in cervical carcinoma worldwide, a total of 8785 cervical carcinomas were tested. The aim was to identify the presence of new HPV sequences as a single genotype in cervical carcinoma specimens with the use of a new testing algorithm, based on the established SPF₁₀ LiPA₂₅ system. However, the 22bp fragment that is used for genotyping in the SPF₁₀ LiPA₂₅ system provides only limited information with respect to the phylogenetic relations between HPV genotypes, and does not allow the characterization of novel HPV genotypes. Therefore the second aim was to obtain additional information of the HPV sequences present in those samples by sequence analysis of DNA segments adjacent to the SPF₁₀ interprimer region.

A segment of 291-bp of the L1 ORF is commonly used for the identification of new papillomavirus types. A new papillomavirus type is recognised if the DNA sequence of the 291-bp fragment of the L1 ORF differs by more than 10% from the closest known type. Differences in homology of between 2% and 10% define a subtype and those of less than 2% define a variant. (Chan et al., J Virol. 1995 May;69(5):3074-83).

### Materials and methods

### Selection of cases

Histological review by two pathologists was carried out on all specimens via the sandwich method to confirm that only biopsies from cervical cancer cases were included. In this method sections for HPV testing are taken from in between sections in which the lesion was confirmed histologically.

### DNA isolation

DNA was released from sections of formalin-fixed, paraffin-embedded tissue by overnight treatment with a proteinase K solution (1 mg/ml). Proteinase K was inactivated at 95°C for 10 min, and 10 µl of (diluted) supernatant was used directly for the SPF₁₀ PCR.

### Detection of novel HPV sequences with the SPF₁₀-LiPA₂₅ system

Broad-spectrum HPV DNA amplification and HPV genotyping was performed using the SPF₁₀-LiPA₂₅ system (SPF₁₀ HPV LiPA, version 1; manufactured by Labo Bio-Medical Products, Rijswijk, The Netherlands). Briefly, SPF₁₀ PCR amplifies a 65-base pair fragment from the L1 region of the HPV genome (Kleter et al., J Clin Microbiol. 1999 Aug;37(8):2508-17). The use of biotinylated reverse primers enabled the capture of the reverse strand of the amplimer onto streptavidin-coated microtiterplates. The captured amplimers were denatured by alkaline treatment, and detected by a defined cocktail of digoxigenin-labeled probes. This method is designated the HPV DEIA, which provides an optical density value and is able to detect more than 50 HPV types (Van Doorn et al., J Clin Microbiol. 2006;44(9):3292-8). Amplimers from positive samples were used to identify 25 individual HPV genotypes (high-risk HPV: 16, 18, 31, 33, 35, 39, 45, 51, 52, 56, 58, 59, 66, 68, 70, and low-risk HPV: 6, 11, 34, 40, 42-44, 53, 54, 74) simultaneously by reverse hybridisation to DNA probes that are attached to nitrocellulose strips in parallel lines (Kleter et al., J Clin Microbiol. 1999 Aug;37(8):2508-17).

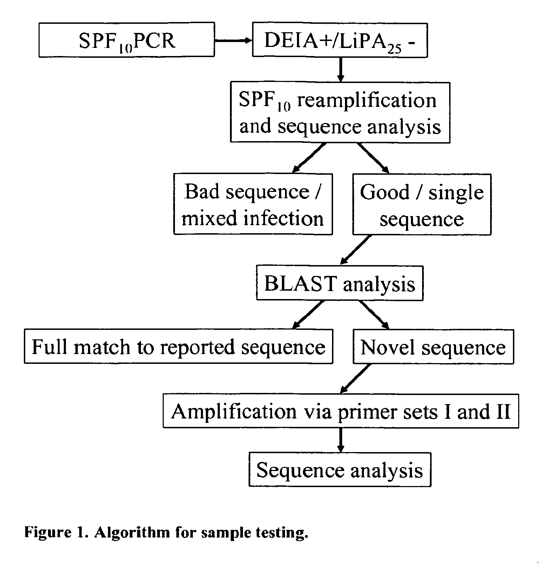

Samples that were determined as HPV positive by the HPV DEIA but yielded no HPV genotype with the LiPA₂₅ were selected for further analysis according to the algorithm presented in Figure 1.

### PCR targets for sequence analysis of DNA segments adjacent to the SPF₁₀ interprimer region

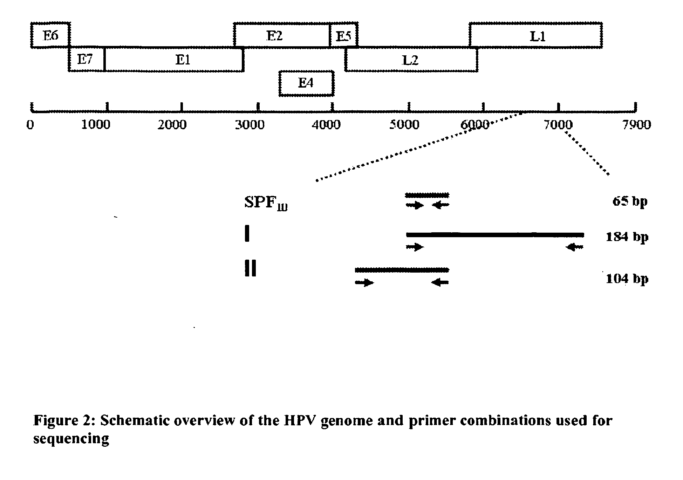

A schematic overview of the HPV genome is presented in Figure 2. Two PCR primer sets were chosen (I and II) to amplify additional fragments of the L1 gene, either upstream or downstream of the 22 bp SPF10 interprimer region. Primer set I comprises forward primers, aimed at positions 6582-6601, and reverse primers, aimed at positions 6741-6765, whereas primer set II comprises forward primers aimed at positions 6543-6565, and reverse primers, aimed at positions 6624-6646. Both PCRs allow for confirmation of the originally found SPF10 sequence.

### PCR conditions for use of the I and II primer set

Briefly, the PCR with primer set I was performed in a final reaction volume of 50 µl, containing 10 µl of the isolated DNA, 3.5 mM MgCl₂, 1X GeneAmp PCR buffer II, 0.2 mM concentrations of deoxynucleoside triphosphates, 7.5 U AmpliTaq Gold DNA polymerase, and primer mix I.

The amplification with primer set II was performed in a final reaction volume of 50 µl, containing 10 µl of the isolated DNA, 3 mM MgCl₂, 1X GeneAmp PCR buffer II, 0.2 mM concentrations of deoxynucleoside triphosphates, 7.5 U AmpliTaq Gold DNA polymerase, and primer mix II.

Both amplifications were performed by a 9-min preheating step at 94°C, followed by 40 cycles of amplification comprising 30 s at 94°C, 45 s at 52°C and 45 s at 72°C. A final elongation step at 72°C of 5 min ends the PCR.

### Sequence analysis

In case of HPV DEIA positive, but LiPA₂₅ negative samples a reamplification of the 65-base pair SPF₁₀ PCR fragment was performed using extended SPF₁₀ primers. The use of extended primers was necessary to elongate the PCR amplimers that were otherwise too short to allow sequencing analysis of the interprimer 22 bp region. Primers annealing to the extended PCR amplimers were used for sequence analysis of the complete SPF₁₀ PCR fragment. All DNA sequencing was carried out according to the manual of the Big Dye Terminator Cycle Sequencing kit (v1) using the ABI3100 *Avant* Genetic Analyzer (Applied Biosystems).

### Results

The sequences that were found were compared to all HPV sequences present in the National Center for Biotechnology Information database utilizing nucleotidenucleotide BLAST (blastn) (http://www.ncbi.nlm.nih.gov/BLAST/). Some of the sequences, although being highly homologous to HPV sequences, did not show a complete match with any reported HPV sequence. At least one novel mismatch was observed in these sequences. These HPV sequences are shown in Figure 3. The HPV reference sequence or the HPV sequence showing the highest degree of homology was used to compare the newly found sequence. If applicable, overlapping sequences obtained from the same sample were assembled.

In particular 98 sequences were identified which came from samples that were DEIA positive but LiPA₂₅ negative, and these sequences comprise some known HPV types and variants thereof which were not previously considered to be high risk types and certain novel variants of known high risk types, as outlined in table 2, along with one novel HPV type.
Figure 1 illustrates the algorithm for sample testing.
Figure 2: Schematic overview of the HPV genome and primer combinations used for sequencing. Various primers sets were used for amplification and subsequent sequence analysis of HPV DNA. The length of the amplimers was calculated for the HPV16 reference sequence with accession number K02718. Primer set I comprises forward primers, aimed at positions 6582-6601, and reverse primers, aimed at positions 6741-6765, whereas primer set II comprises forward primers aimed at positions 6543-6565, and reverse primers, aimed at positions 6624-6646.
Figure 3: Alignment of new HPV sequences found in cervical carcinoma. Sequences were derived from sequence analysis using various primer combinations. If applicable, overlapping sequences obtained from the same sample were combined. These sequences were aligned with their phylogeneticaly most related reference type or isolate (as determined by BLAST analysis), which were obtained from Genbank (accession number in parenthesis; no complete reference genome is defined for HPV type 64, the sequence of a clinical isolate was used). SPXCC015 and SPXCC019 were found only once and sequencing of a longer fragment succeeded. In this figure they are designated as HPV16var2 and HPV18var1, respectively.

### Discussion

HPV sequences not yet published on Genbank were found as single infection in cervical cancer. These results might open a new discussion towards the focus of current HPV diagnostics and limitations of present assays and studies. Future HPV assays should incorporate these newly found HPV sequences because of their carcinogenic potential.

**Table 1**

| SEQ ID NO | Fig 3 cross reference | Start - end position (accession number) | Discriminatory nucleotide position | sequence |
|---|---|---|---|---|
| 1 | SPXCC004 | 6602-6623 (K02718) | 6621 | CATTTGTTGGGGTAACCAAtTA |
| 2 | HPV16var2 | 6602-6740 (K02718) | 6620 | CATTTGTTGGGGTAACCAcCTATTTGTTACTGTTGTTGATACTACACGCAGTACAAATATGTCATTATGT GCTGCCATATCTACTTCAGAAACTACATATAAAAATACTAACTTTAAGGAGTACCTACGACATGGGGAG |
| 3 | SPXCC011 | 6602-6623 (K02718) | 6620 | CATTTGTTGGGGTAACCAgCTA |
| 4 | HPV16var4 | 6566-6623 (K02718) | 6620 | TTATTGGTTACAACGAGCACAGGGCCACAATAATGGCATTTGTTGGGGTAACCAgCTA |
| 5 | HPV18var1 | 6552-6693 (EF202155) | 6555 | TATtTGCTGGCATAATCAATTATTTGTTACTGTGGTAGATACCACTCGTAGTACCAATTTAACAATATGTG CTTCTACACAGTCTCCTGTACCTGGGCAATATGATGCTACCAAATTTAAGCAGTATAGCAGACATGTTGAA |
| 6 | HPV39var1 | 6569-6746 (M62849) | 6624 & 6638 | TTATTGGCTACATAAGGCCCAGGGCCACAACAATGGTATATGTTGGCATAATCAAgTATTTCTTACTGTa GTGGACACTACCCGTAGTACCAACTTnnCATTATCTACCTCTATAGAGTCTTCCATACCTTCTACATATGA TCCTTCTAAGTTTAAGGAATATACCAGGCACGTGGAG , |
| 7 | SPXCC06 | 6605-6626 (M62849) | 6624 | TATATGTTGGCATAATCAAgTA |
| 8 | HPV68var1 | 2582-2723 (M73258, partial sequence) | 2596 | TATTTGTTGGCATAATgAATTATTTCTTACTGTTGTGGATACCACTCGCAGTACCAATTTTACTTTGTCT ACTACTACTGAATCAGCTGTACCAAATATTTATGATCCTAATAAATTTAAGGAATATATTAGGCATGTTGA G |
| 9 | SPXCC008 | 6559-6580 (X7483) | 6578 | CATTTGCTGGGGTAATCAAgTA |
| 10 | HPV56var1 | 6559-6694 (X74483) | 6578 | CATTTGCTGGGGTAATCAAgTATTTGTTACTGTAGTAGATACTACTAGAAGTACTAACATGACTATTAGT ACTGCTACAGAACAGTTAAGTAAATATGATGCACGAAAAATTAATCAGTACCTTAGACATGTGGAG |
| 11 | HPV56var2 | 6559-6694 (X74483) | 6578 & 6595 | CATTTGCTGGGGTAATCAAgTATTTGTTACTGTAGTgGATACTACTAGAAGTACTAACATGACTATTA |
| | | | | GTACTGCTACAGAACAGTTAAGTAAATATGATGCACGAAAAATTAATCAGTACCTTAGACATGTGGAG |
| 12 | HPV56var3 | 6523-6580 (X74483) | 6578 | TTATTGGTTGCAACGTGCCCAAGGCCATAATAATGGCATTTGCTGGGGTAATCAAgTA |
| 13 | SPXCC002 | 6584-6605 (D21208) | 6603 | TATATGCTGGGGTAATCAAcTA |
| 14 | HPV67var1 | 6584-6719 (D21208) | 6603 & 6697 | TATATGCTGGGGTAATCAAcTATTTGTTACTGTTGTAGACACTACACGTAGTACCAACATGACTTTAT GTTCTGAGGAAAAATCAGAGGCTACATACAAAAATGAAAACTTTAgGGAATACCTTAGACATGTGGAA |
| 15 | HPV67var2 | 6548-6719 (D21208) | 6569 & 6603 | ATATTGGTTACAACGCGCACAgGGTCATAACAATGGTATATGCTGGGGTAATCAAcTATTTGTTACTGT TGTAGACACTACACGTAGTACCAACATGACTTTATGTTCTGAGGAAAAATCAGAGGCTACATACAAAAAT GAAAACTTTAAGGAATACCTTAGACATGTGGAA |
| 16 | SPXCC003 | 6617-6638 (X74474) | 622 | CATTTaTTGGGGCAACCAGGTA |
| 17 | SPXCC007 | 6553-6574 (X74472) | 6559 | TATCTGcTGGGGCAATCAATTG |
| 18 | SPXCC009 | 21-42 (U12495, partial sequ | 26 & 40 | AATTTaTTGGCATAATCAAtTG |
| 19 | SPXCC005 | 6529-6550 (AF436129) | 6539 & 6548 & 6550 | TATTTGTTGGaGCAATCAAaTa |
| 20 | SPXCC018 | 33 nt before pos. 1-118 AJ0 | | TatttgctggtttaatcaattgtttgttacagtTGTGGATACCACTAGAAGTACCAATTTTACTATTAG TGCTGCTACCCAGGCCTCCGCTACCCAGGCCTCCACGGAATATAAGTCTACTAATTTTAAGGAATATCTT AGGCATGTGGAG |
| 21 | HPV30var1 | 6662-6720 (X74474) | 6689 | TAGGAACACAAACATGACTATATCTGCcACCACACAAACGTTATCCACATATAATTCAA |
| 22 | HPV30var2 | 6689-6720 (X74474) | 6714 | AACCACACAAACGTTATCCACATATgATTCAA |
| 23 | HPV16var3 | 6602-6740 (K02718) | 6617 | CATTTGTTGGGGTAAtCAACTATTTGTTACTGTTGTTGATACTACACGCAGTACAAATATGTCATTATG TGCTGCCATATCTACTTCAGAAACTACATATAAAAATACTAACTTTAAGGAGTACCTACGACATGGGGAG |
| 24 | SPXCC021 | 6464-6485 (X74476) | 6469 | CATTTaCTGGCATAATCAACTG |
| 25 | SPXCC022 | 6561-6582 (U37488) | 6580 | TATTTGTTGGGGCAATCAGtTG |
| 26 | SPXCC023 | 21-42 (U12495, partial sequ | 21 | cATTTGTTGGCATAATCAACTG |
| 27 | SPXCC016 | 2582-2603 (M73258, partial sequence) | 2598 | TATTTGTTGGCATAATgAATTA |
| 28 | SPXCC024 | 6617-6638 (X74474) | 6635 | CATTTGTTGGGGCAACCAaGTA |
| 29 | HPV82var1 | 6500-6677 (AB027021) | 6583 & 6590 & 6607 & 6619 | TTATTGGTTGCATCGCGCCCAGGGCCACAATAATGGCATTTGCTGGAATAATCAGCTTTTTATTACTTG TGTTGACACTACTAgAAGTACtAATTTAACCATTAGCAtTGCTGTTACTCaATCTGTTGCACAAACATTT ACTCCAGCAAACTTTAAGCAGTACATTAGGCATGGGGAA |
| 30 | HPV91var1 | 6794-6971 (AF419318) | 6818 & 6833 | GCTTTGGATGCAAAAAGCACAGGGaCATAATAACGGCATtTGTTTTGGTAACCAGGTGTTTGTAACTGTT GTGGATACAACTCGCAGCACTAACTTAACCTTGTGTGCATCCACTGAGTCTGTGCTACCTACTACATATG ACAACACAAAGTTCAAAGAATATTTAAGGCATGCAGAA |

| | | | | |
|---|---|---|---|---|
| SEQ ID NO 20 is an isolated HPV polynucleotide sequence, which comprises a region of 33 novel nucleotides upstream of position 1 when compared to the unclassified HPV reference sequence AJ012757. The remaining part of this polynucleotide sequence is homologous to position 1-118 of the reference sequence AJ012757. Blast analysis restricted to the first 33 nucleotides of SEQ ID NO 20 (SEQ ID NO 20*) revealed three discriminatory nucleotides with reference sequence AJ400628.:Nucleotides indicated by lower case letters in the table above are also referred to herein as 'discriminatory' nucleotides | | | | |

**Table 2 Overview of single infections with HPV types or novel sequences found in cervical carcinoma**

| **Detection of single HPV sequences in cervical carcinoma** | | |
|---|---|---|
| **HPV type or novel HPV sequence** | **Number of different novel sequences** | **Number of samples in which the sequence was found** |
| **HPV16vars** | **5** | |
| **HPV18vars** | **1** | |
| **HPV39vars** | **2** | |
| **HPV56vars** | **4** | |
| **HPV68vars** | **2** | |
| **HPV67vars** | **3** | |
| **HPV30vars** | **4** | |
| **HPV26vars** | **1** | |
| **HPV64vars** | **2** | |
| **HPV34vars** | **1** | |
| **HPV54vars** | **2** | |
| **HPV82vars** | **1** | |
| **HPV91vars** | **1** | |
| **Uncl. HPV** | **1** | |
| **HPV67** | | **6** |
| **HPV30** | | **28** |
| **HPV26** | | **23** |
| **HPV20** | | **1** |
| **HPV61** | | **1** |
| **HPV69** | | **9** |
| **Total** | **30** | **68** |

## Claims

1. An *in vitro* use of a probe specific for HPV type 69 in the screening of tissue samples for the identification of a risk of development of cervical cancer, wherein detection of HPV 69 is indicative of high risk.

2. Use according to claim 1 wherein the probe is complementary to a part of the HPV L1, E1, E6 or E7 gene.

3. Use according to claim 1 or 2 wherein the probe is used in conjunction with a second probe specific for a high risk type, under the same reaction conditions.

4. Use according to claim 3 wherein the second probe is selected from the list of probes specific for high risk HPV types 20, 61, 30, 67 and 91, or specific for the variants of HPV 64, 34, or 54 listed in table 1.

5. Use according to claim 3 wherein the second probe is specific for one of HPV 16, 18, 26, 31, 33, 35, 39, 45, 51, 52, 56, 58, 59, 68, 73 and 82.

6. An *in vitro* use of a kit comprising a probe specific for HPV type 69 and additionally comprising a probe specific for an HPV high risk type selected from HPV 16, 18, 23, 31, 33, 35, 39, 45, 51, 52, 53, 56, 58, 59, 66, 68, 73 and 82 in the screening of tissue samples for the identification of a risk of development of cervical cancer, wherein detection of HPV 69 is indicative of high risk.

7. Use according to claim 6 wherein the kit additionally comprises a means to detect a second HPV type selected from the list of HPV types 20, 61, 30, 67 and 91, or a means to detect the variants of HPV 64, 34, or 54 listed in table 1.

8. Use of HPV type 69 in the preparation of a vaccine for prevention or treatment of cervical cancer.

9. A vaccine for the prevention of cervical cancer comprising an HPV antigen, or nucleic acid encoding said antigen, wherein the HPV antigen is HPV type 69, wherein vaccination results in immunity against HPV type 69.

10. A vaccine according to claim 9 wherein the antigen is an L1 antigen or part thereof, or nucleic acid encoding said antigen or part thereof.

11. A vaccine according to claim 9 or 10 wherein the vaccine additionally comprises an antigen from a HPV high risk type selected from the list consisting of HPV 16, 18, 26, 31, 33, 35, 39, 45, 51, 52, 53, 56, 58, 59, 66, 68, 73 and 82, or nucleic acid encoding said antigen.

## Patentansprüche

1. *In-vitro*-Verwendung einer für HPV-Typ 69 spezifischen Sonde beim Screening von Gewebeproben zur Identifikation eines Risikos für die Entwicklung von Zervixkrebs, wobei der Nachweis von HPV 69 auf ein hohes Risiko hindeutet.

2. Verwendung nach Anspruch 1, wobei die Sonde zu einem Teil des HPV-L1-, -E1-, -E6- oder -E7-Gens komplementär ist.

3. Verwendung nach Anspruch 1 oder 2, wobei die Sonde, unter den gleichen Reaktionsbedingungen, zusammen mit einer zweiten für einen Hochrisikotyp spezifischen Sonde verwendet wird.

4. Verwendung nach Anspruch 3, wobei die zweite Sonde aus der Liste von Sonden ausgewählt ist, die für Hochrisiko-HPV-Typen 20, 61, 30, 67 und 91 spezifisch sind oder für die in Tabelle 1 aufgelisteten Varianten von HPV 64, 34 oder 54 spezifisch sind.

5. Verwendung nach Anspruch 3, wobei die zweite Sonde für eines von HPV 16, 18, 26, 31, 33, 35, 39, 45, 51, 52, 56, 58, 59, 68, 73 und 82 spezifisch ist.

6. *In-vitro*-Verwendung eines Kits, das eine für HPV-Typ 69 spezifische Sonde umfasst und zusätzlich eine Sonde umfasst, die für einen HPV-Hochrisikotyp spezifisch ist, ausgewählt aus HPV 16, 18, 23, 31, 33, 35, 39, 45, 51, 52, 53, 56, 58, 59, 66, 68, 73 und 82, beim Screening von Gewebeproben zur Identifikation eines Risikos für die Entwicklung von Zervixkrebs, wobei der Nachweis von HPV 69 auf ein hohes Risiko hindeutet.

7. Verwendung nach Anspruch 6, wobei das Kit zusätzlich ein Mittel zum Nachweis eines zweiten aus der Liste von HPV-Typen 20, 61, 30, 67 und 91 ausgewählten HPV-Typs oder ein Mittel zum Nachweis der Varianten von in Tabelle 1 aufgelisteten HPV 64, 34 oder 54 umfasst.

8. Verwendung von HPV-Typ 69 bei der Herstellung eines Impfstoffs zur Prävention oder Behandlung von Zervixkrebs.

9. Impfstoff zur Prävention von Zervixkrebs, umfassend ein HPV-Antigen oder eine für das Antigen kodierende Nukleinsäure, wobei das HPV-Antigen HPV-Typ 69 ist, wobei die Impfung zur Immunität gegen HPV-Typ 69 führt.

10. Impfstoff nach Anspruch 9, wobei das Antigen ein L1-Antigen oder ein Teil davon oder eine für das Antigen oder einen Teil davon kodierende Nukleinsäure ist.

11. Impfstoff nach Anspruch 9 oder 10, wobei der Impfstoff zusätzlich ein Antigen von einem HPV-Hochrisikotyp, ausgewählt aus der Liste, bestehend aus HPV 16, 18, 26, 31, 33, 35, 39, 45, 51, 52, 53, 56, 58, 59, 66, 68, 73 und 82, oder eine für das Antigen kodierende Nukleinsäure umfasst.

## Revendications

1. Utilisation *in vitro* d'une sonde spécifique pour le HPV de type 69 dans l'analyse d'échantillons tissulaires pour l'identification d'un risque de développement d'un cancer cervical, dans laquelle la détection du HPV 69 est indicatrice d'un risque élevé.

2. Utilisation selon la revendication 1, dans laquelle la sonde est complémentaire d'une partie du gène L1, E1, E6 ou E7 du HPV.

3. Utilisation selon la revendication 1 ou 2, dans laquelle la sonde est utilisée conjointement avec une deuxième sonde spécifique pour un type à risque élevé, dans les mêmes conditions réactionnelles.

4. Utilisation selon la revendication 3, dans laquelle la deuxième sonde est choisie dans la liste des sondes spécifiques pour les HPV des types 20, 61, 30, 67 et 91 à risque élevé, ou spécifiques pour les variants du HPV 64, 34 ou 54 énumérés dans le tableau 1.

5. Utilisation selon la revendication 3, dans laquelle la deuxième sonde est spécifique pour l'un des HPV 16, 18, 26, 31, 33, 35, 39, 45, 51, 52, 56, 58, 59, 68, 73 et 82.

6. Utilisation *in vitro* d'un kit comprenant une sonde spécifique pour le HPV de type 69 et comprenant en outre une sonde spécifique pour un HPV d'un type à risque élevé choisi parmi les HPV 16, 18, 23, 31, 33, 35, 39, 45, 51, 52, 53, 56, 58, 59, 66, 68, 73 et 82 dans l'analyse d'échantillons tissulaires pour l'identification d'un risque de développement d'un cancer cervical, dans laquelle la détection du HPV 69 est indicatrice d'un risque élevé.

7. Utilisation selon la revendication 6, dans laquelle le kit comprend en outre un moyen pour détecter un second type de HPV choisi dans la liste des HPV des types 20, 61, 30, 67 et 91, ou un moyen pour détecter les variants des HPV 64, 34 ou 54 énumérés dans le tableau 1.

8. Utilisation du HPV du type 69 dans la préparation d'un vaccin destiné à la prévention ou au traitement d'un cancer cervical.

9. Vaccin destiné à la prévention d'un cancer cervical comprenant un antigène de HPV, ou un acide nucléique codant pour ledit antigène, dans lequel l'antigène de HPV est le HPV de type 69, dans lequel la vaccination entraîne une immunité contre le HPV de type 69.

10. Vaccin selon la revendication 9, dans lequel l'antigène est un antigène L1 ou une partie de celui-ci, ou un acide nucléique codant pour ledit antigène ou ladite partie de celui-ci.

11. Vaccin selon la revendication 9 ou 10, dans lequel le vaccin comprend en outre un antigène provenant d'un HPV d'un type à risque élevé choisi dans la liste constituée des HPV 16, 18, 26, 31, 33, 35, 39, 45, 51, 52, 53, 56, 58, 59, 66, 68, 73 et 82, ou un acide nucléique codant pour ledit antigène.
